# EUROPEAN PATENT APPLICATION

(11) **EP 2 468 723 A1**
(43) Date of publication of application: **27.06.2012**
(21) Application number: 10810002.5
(22) Date of filing: 19.08.2010
(51) Int. Cl.: C07C 407/00, C07C 409/12

(54) **METHOD FOR HANDLING AN AQUEOUS SOLUTION CONTAINING A WATER-SOLUBLE SUBSTANCE, METHOD FOR MANUFACTURING ALKYLBENZENE HYDROPEROXIDES, AND METHOD FOR MANUFACTURING HYDROXYBENZENES**

(30) Priority: 19.08.2009 JP 2009189744; 27.11.2009 JP 2009269709
(71) Applicant: Sumitomo Chemical Company, Limited, Tokyo 104-8260 (JP)
(72) Inventor: ONUMA, Mitsuru, Ichihara-shi Chiba 299-0117 (JP); NORITAKE, Tomoyuki, Ichihara-shi Chiba 290-0035 (JP); GOTO, Shigeru, Chiba-shi Chiba 262-0032 (JP)
(74) Representative: Duckworth, Timothy John
(86) International application number: PCT/JP2010/063996
(87) International publication number: WO 2011/021661

(57) **Abstract**

There is provided a method for handling an aqueous solution containing a water-soluble substance that changes into a water-insoluble substance over time, including a step of bringing the aqueous solution to be treated containing the water-soluble substance that changes into the water-insoluble substance over time into contact with a water-insoluble organic solvent, and extracting the water-soluble substance into the water-insoluble organic solvent. Preferably, the water-soluble substance is alkylbenzene hydroperoxide, and the water-insoluble substance is alkylbenzene hydroperoxide-derived alcohol. Preferably, the aqueous solution to be treated is an alkaline aqueous phase separated in a water-washing step in a process of manufacturing the alkylbenzene hydroperoxide that has an alkali extraction step, the water-washing step, an oxidation step, and a reaction solution separation step.

## Description

### TECHNICAL FIELD

The present invention relates to a method for handling an aqueous solution containing a water-soluble substance, a method for manufacturing alkylbenzene hydroperoxide, and a method for manufacturing hydroxybenzene. More specifically, the present invention relates to a method for handling an aqueous solution containing a water-soluble substance, a method for manufacturing alkylbenzene hydroperoxide, and a method for manufacturing hydroxybenzene, which have such excellent features that prevention of problems such as blockage in an apparatus caused by precipitation of a water-insoluble substance as well as long-term stable operation are possible.

### BACKGROUND ART

Alkylbenzene hydroperoxide is a compound useful as an intermediate of hydroxybenzene. As a manufacturing method therefor, Japanese Patent Laying-Open No. 2007-246511 (PTL 1), for example, describes a manufacturing method of causing dialkylbenzene to undergo an oxidation reaction to obtain an oxidation reaction solution, separating desired alkylbenzene hydroperoxide by extracting the obtained oxidation reaction solution using an alkaline aqueous solution, and thereafter, water-washing an oil phase containing unreacted dialkylbenzene and recycling the oil phase into an oxidation step. Normally, an aqueous phase obtained after water-washing the oil phase containing the unreacted dialkylbenzene is discharged outside the system.

### CITATION LIST

### PATENT LITERATURE

PTL 1: Japanese Patent Laying-Open No. 2007-246511

### SUMMARY OF INVENTION

### TECHNICAL PROBLEM

The above-mentioned aqueous phase obtained after water-washing the oil phase containing the unreacted dialkylbenzene contains alkalis. Therefore, there has been a problem described below. Specifically, a part of the alkylbenzene hydroperoxide present in the oil phase moves to the aqueous phase, and the alkylbenzene hydroperoxide that has moved to the aqueous phase changes into alkylbenzene hydroperoxide-derived alcohol over time and is precipitated, and the precipitated water-insoluble substance produces blockage and the like in an apparatus.

Under these circumstances, a problem to be solved by the present invention is to provide a method for handling an aqueous solution containing a water-soluble substance, a method for manufacturing alkylbenzene hydroperoxide, and a method for manufacturing hydroxybenzene, which have such excellent features that prevention of problems such as blockage in an apparatus caused by precipitation of a water-insoluble substance as well as long-term stable operation are possible.

### SOLUTION TO PROBLEM

The present invention is directed to a method for handling an aqueous solution containing a water-soluble substance that changes into a water-insoluble substance over time, including the step of bringing the aqueous solution containing the water-soluble substance that changes into the water-insoluble substance over time into contact with a water-insoluble organic solvent, and extracting the water-soluble substance into the water-insoluble organic solvent.

The present invention is also directed to a method for manufacturing alkylbenzene hydroperoxide, having an alkali extraction step, a water-washing step, an oxidation step, a reaction solution separation step, and a recovery step described below. alkali extraction step: a step of bringing a material solution containing alkylbenzene, phenol and alkylbenzene hydroperoxide into contact with an alkaline aqueous solution, and thereafter, performing separation into a first oil phase containing an alkaline substance and a first alkaline aqueous phase containing at least a part of the phenol and at least a part of the alkylbenzene hydroperoxide water-washing step: a step of bringing the first oil phase into contact with water, extracting at least a part of the alkylbenzene hydroperoxide and at least a part of the alkaline substance into the water from the first oil phase, and thereafter, performing separation into a second oil phase and a second alkaline aqueous phase containing the alkylbenzene hydroperoxide oxidation step: a step of oxidizing the second oil phase using a gas containing air or oxygen, with the second oil phase being in contact with an alkaline aqueous phase, and obtaining a reaction solution containing the alkylbenzene hydroperoxide reaction solution separation step: a step of performing separation into a third alkaline aqueous phase and a third oil phase containing the alkylbenzene hydroperoxide by oil-water separation of the reaction solution obtained in the oxidation step recovery step: a step of bringing the second alkaline aqueous phase into contact with a water-insoluble organic solvent, performing separation into an aqueous phase and a fourth oil phase containing at least a part of the alkylbenzene hydroperoxide extracted into the water-insoluble organic solvent, and discharging the aqueous phase outside a system

Preferably, in the method for manufacturing alkylbenzene hydroperoxide according to the present invention, the alkylbenzene is alkylbenzene having at least one isopropyl group, and the alkylbenzene hydroperoxide is alkylbenzene hydroperoxide having at least one 2-hydroperoxy-2-propyl group.

The present invention is also directed to a method for manufacturing hydroxybenzene, including a decomposition step described below. decomposition step: a step of decomposing, under presence of an acid catalyst, alkylbenzene hydroperoxide having at least one 2-hydroperoxy-2-propyl group obtained by the above-mentioned method for manufacturing alkylbenzene hydroperoxide having at least one 2-hydroperoxy-2-propyl group, and obtaining the hydroxybenzene.

### ADVANTAGEOUS EFFECTS OF INVENTION

According to the present invention, prevention of problems such as blockage in an apparatus caused by precipitation of a water-insoluble substance as well as long-term stable operation are possible.

### BRIEF DESCRIPTION OF DRAWINGS

Fig. 1 is a schematic view of a process flow in Example 1 for manufacturing alkylbenzene hydroperoxide.
Fig. 2 is a schematic view of a process flow in Comparative Example 1 for manufacturing alkylbenzene hydroperoxide.

### DESCRIPTION OF EMBODIMENTS

An embodiment of the present invention will be described in detail hereinafter.

### 1. Method for handling aqueous solution containing water-soluble substance that changes into water-insoluble substance over time

A method for handling an aqueous solution to be treated containing a water-soluble substance that changes into a water-insoluble substance over time according to the present invention includes a step of bringing the aqueous solution to be treated containing the water-soluble substance that changes into the water-insoluble substance over time into contact with a water-insoluble organic solvent, and extracting the water-soluble substance into the water-insoluble organic solvent.

The water-soluble substance that changes into the water-insoluble substance over time in the present invention refers to a substance (water-soluble substance) that forms an aqueous solution with the original substance dissolved in an aqueous phase, and a compound having a feature of a substance (water-insoluble substance) that has such properties that at least a part thereof decomposes into a different compound with the passage of time, that the solubility of the changed compound in the aqueous phase is lower than that of the original water-soluble substance, and that crystals are precipitated in the aqueous phase. For example, the water-soluble substance that changes into the water-insoluble substance over time refers to a substance whose mol conversion rate from the water-soluble substance to the water-insoluble substance after 72-hour storage in the aqueous solution is 1% or more. The mol conversion rate after 72-hour storage refers to a ratio between the number of moles of the initial water-soluble substance and the number of moles of the water-soluble substance that has changed into the water-insoluble substance after a lapse of 72 hours.

Specifically, the water-soluble substance that changes into the water-insoluble substance over time includes alkylbenzene hydroperoxide and the like, preferably ethylbenzene hydroperoxide, cumene hydroperoxide, o-diisopropylbenzene dihydroperoxide (hereinafter, may be referred to as "o-DHPO"), 2-(2-hydroxy-2-propyl)-1-(2-hydroperoxy-2-propyl)benzene (hereinafter, may be referred to as "o-CHPO"), m-diisopropylbenzene dihydroperoxide(1,3-di-(2-hydroperoxy-2-propyl)benzene (hereinafter, may be referred to as "m-DHPO"), 3-(2-hydroxy-2-propyl)-1-(2-hydroperoxy-2-propyl)benzene (hereinafter, may be referred to as "m-CHPO"), p-diisopropylbenzene dihydroperoxide (hereinafter, may be referred to as "p-DHPO"), or 4-(2-hydroxy-2-propyl)-1-(2-hydroperoxy-2-propyl)benzene (hereinafter, may be referred to as "p-CHPO"), more preferably o-DHPO, o-CHPO, m-DHPO, m-CHPO, p-DHPO, or p-CHPO, further preferably o-CHPO, m-CHPO or p-CHPO, and particularly preferably m-CHPO. The water-soluble substance that changes into the water-insoluble substance over time may be present in the aqueous solution to be treated alone or in a mixture of two or more types.

If the above-mentioned water-soluble substance is alkylbenzene hydroperoxide, the water-insoluble substance is alkylbenzene hydroperoxide-derived alcohol. If the above-mentioned water-soluble substance is o-DHPO or o-CHPO, the water-insoluble substance is 1,2-di-(2-hydroxy-2-propyl)benzene (hereinafter, may be referred to as "o-DCA"). If the above-mentioned water-soluble substance is m-DHPO or m-CHPO, the water-insoluble substance is 1,3-di-(2-hydroxy-2-propyl)benzene (hereinafter, may be referred to as "m-DCA"). If the above-mentioned water-soluble substance is p-DHPO or p-CHPO, the water-insoluble substance is 1,4-di-(2-hydroxy-2-propyl)benzene (hereinafter, may be referred to as "p-DCA"). Particularly if the aqueous solution to be treated is an alkaline aqueous solution such as a sodium hydroxide aqueous solution, a sodium carbonate aqueous solution, a potassium hydroxide aqueous solution, and a potassium carbonate aqueous solution, decomposition of the above-mentioned alkylbenzene hydroperoxide is promoted. Therefore, this is suitable for application of the present invention.

From the viewpoint of extraction efficiency and solvent recovery, the water-insoluble organic solvent is preferably ketone having a carbon number of 4 to 10, ether having a carbon number of 4 to 10, or alcohol having a carbon number of 4 to 8, and more preferably methyl isobutyl ketone. The water-insoluble organic solvent may be used alone or in a mixture of two or more types.

From the viewpoint of extraction at a desired level of the water-soluble substance that changes into the water-insoluble substance over time as well as reduction in cost such as thermal energy in purifying the solvent, the amount of the water-insoluble organic solvent used is preferably 1 to 200 parts by weight, more preferably 10 to 100 parts by weight, and further preferably 20 to 50 parts by weight with respect to 100 parts by weight of the aqueous solution containing the water-soluble substance that changes into the water-insoluble substance over time.

The contact operation can be performed using normal equipment and under normal operation conditions. An apparatus can include, for example, a mixer settler, an extraction column and the like. Extraction conditions can include, for example, a temperature of 20 to 80°C.

The aqueous phase obtained by contact with the water-insoluble organic solvent is discarded. A discarding method includes, for example, an incineration treatment method by a submerged combustion apparatus. The submerged combustion apparatus is an apparatus spraying a waste liquid containing a high concentration of a COD component in a high-temperature atmosphere of 800 to 1200°C, oxidatively decomposing the waste liquid, and thereby rendering the waste liquid completely harmless. The waste liquid sprayed into a furnace that is kept at a high temperature by an auxiliary burner is oxidized and rendered completely harmless. Furthermore, a salt contained in the waste liquid is rapidly cooled and collected in a cooling can. In addition, a metal is collected as an oxide. Small-capacity to large-capacity incineration systems using the submerged combustion method are used in many industrial fields.

The aqueous solution to be treated containing the water-soluble substance that changes into the water-insoluble substance over time includes, for example, the aqueous phase and the like obtained by oxidizing alkylbenzene with a gas containing air or oxygen to obtain a reaction solution containing alkylbenzene hydroperoxide, and water-washing an oil phase obtained after separation and recovery of the alkylbenzene hydroperoxide from the reaction solution. An oxidation method includes, for example, auto-oxidation with an oxygen-containing gas such as air and oxygen-concentrated air. This oxidation reaction may be produced without using an additive, or an additive such as alkali may be used. The normal reaction temperature is 50 to 200°C and the reaction pressure is between the atmospheric pressure and 5 MPa. In the case of the oxidation method using the additive, an alkali metal compound such as sodium hydroxide and potassium hydroxide, an alkali earth metal compound, or an alkali metal carbonate such as sodium carbonate and sodium hydrogen carbonate, or ammonia and an ammonium carbonate, an alkali metal ammonium carbonate, or the like is used as an alkaline reagent.

The aqueous solution to be treated containing the water-soluble substance that changes into the water-insoluble substance over time is preferably a second alkaline aqueous phase obtained in a process of manufacturing the alkylbenzene hydroperoxide as described below. This manufacturing process has an alkali extraction step, a water-washing step, an oxidation step, and a reaction solution separation step. alkali extraction step: a step of bringing a material solution containing alkylbenzene, phenol and alkylbenzene hydroperoxide into contact with an alkaline aqueous solution, and thereafter, performing separation into a first oil phase containing an alkaline substance and a first alkaline aqueous phase containing at least a part of the phenol and at least a part of the alkylbenzene hydroperoxide
water-washing step: a step of bringing the first oil phase into contact with water, extracting at least a part of the alkylbenzene hydroperoxide and at least a part of the alkaline substance into the water from the first oil phase, and thereafter, performing separation into a second oil phase and a second alkaline aqueous phase containing the alkylbenzene hydroperoxide
oxidation step: a step of oxidizing the second oil phase using a gas containing air or oxygen, with the second oil phase being in contact with an alkaline aqueous phase, and obtaining a reaction solution containing the alkylbenzene hydroperoxide reaction solution separation step: a step of performing separation into a third alkaline aqueous phase and a third oil phase containing the alkylbenzene hydroperoxide by oil-water separation of the reaction solution obtained in the oxidation step

The alkali extraction step is the step of bringing the material solution containing alkylbenzene, phenol and alkylbenzene hydroperoxide into contact with the alkaline aqueous solution, and thereafter, performing separation into the first oil phase containing the alkaline substance and the first alkaline aqueous phase containing at least a part of the phenol and at least a part of the alkylbenzene hydroperoxide. The alkylbenzene herein refers to a substance serving as a material in manufacturing the alkylbenzene hydroperoxide in the oxidation step. The phenol herein refers to a substance by-produced as an impurity in the oxidation step, or an impurity generated from the substances changed in each step.

If the alkylbenzene hydroperoxide manufactured in the oxidation step is:
(1) o-DHPO, the alkylbenzene is o-diisopropylbenzene, and the phenol is, for example, o-isopropylphenol, o-isopropenylphenol, o-hydroxy acetophenone or the like;
(2) m-DHPO, the alkylbenzene is m-diisopropylbenzene, and the phenol is, for example, m-isopropylphenol, m-isopropenylphenol, m-hydroxy acetophenone or the like; and
(3) p-DHPO, the alkylbenzene is p-diisopropylbenzene, and the phenol is, for example, p-isopropylphenol, p-isopropenylphenol, p-hydroxy acetophenone or the like.

The alkaline aqueous solution includes, for example, a sodium hydroxide aqueous solution, a potassium hydroxide aqueous solution, a sodium carbonate aqueous solution and the like, and preferably is a sodium hydroxide aqueous solution. The concentration of alkalis in the alkaline aqueous solution is preferably 5 to 10 weight % from the viewpoint of extraction efficiency.

The extraction temperature is not particularly limited. However, excessively high temperature is not preferable because the alkylbenzene hydroperoxide decomposes thermally, and excessively low temperature is not preferable because the extraction efficiency may decrease and the solution separation property may become worse. Therefore, the normal extraction operation temperature is 10 to 80°C, and preferably 20 to 60°C. A container used in the extraction operation is not particularly limited. The container can, however, include, for example, a combination of a settling drum having the gravity separation function and a portion having the mixing function such as a line mixer and a stirring bath, and the like. When the state of mixed oil and water is good depending on the solution composition, the stirring function may be omitted. By bringing the solution containing the alkylbenzene, the phenol and the alkylbenzene hydroperoxide into contact with the alkaline aqueous solution, at least a part of the phenol moves from the solution to the alkaline aqueous solution side. Similarly, at least a part of the alkylbenzene hydroperoxide also moves to the alkaline aqueous solution side. The first oil phase obtained by oil-water separation is then sent to the water-washing step because the first oil phase contains the alkaline substance due to mixing of the alkaline aqueous solution.

The alkaline aqueous phase obtained in the alkali extraction step contains the alkylbenzene hydroperoxide, and if the alkylbenzene hydroperoxide is alkylbenzene hydroperoxide having at least one 2-hydroperoxy-2-propyl group, the alkylbenzene hydroperoxide can be used as a material in manufacturing corresponding hydroxybenzene.

The water-washing step is the step of bringing the first oil phase containing the alkaline substance, which was obtained in the alkali extraction step, into contact with water, extracting at least a part of the alkylbenzene hydroperoxide and at least a part of the alkaline substance into the water from the first oil phase, and thereafter, performing separation into the second oil phase and the second alkaline aqueous phase containing the alkylbenzene hydroperoxide. From the viewpoint of removing the alkaline substance in the first oil phase, water that does not substantially contain the alkaline substance, or process water having a lower concentration of the alkaline substance than that of the alkaline aqueous solution used in the alkali extraction step can be used in water-washing. For example, from the viewpoint of reducing discharged water, it is preferable to recycle the third alkaline aqueous phase obtained in the reaction solution separation step as at least a past of the water-washing step.

The water-washing temperature is not particularly limited. However, excessively high temperature is not preferable because the alkylbenzene hydroperoxide decomposes thermally, and excessively low temperature is not preferable because the extraction efficiency may decrease and the solution separation property may become worse. Therefore, the normal water-washing operation temperature is 10 to 80°C, and preferably 20 to 60°C. A container used in the water-washing operation is not particularly limited. The container can, however, include, for example, a combination of a settling drum having the gravity separation function and a portion having the mixing function such as a line mixer and a stirring bath, and the like. When the state of mixed oil and water is good depending on the solution composition, the stirring function may be omitted. By bringing the second oil phase containing the alkaline substance into contact with water, the alkaline substance mixed into the second oil phase moves to the aqueous phase. Similarly, at least a part of the alkylbenzene hydroperoxide also moves to the aqueous phase.

The oxidation step is the step of oxidizing the second oil phase obtained in the water-washing step using a gas containing air or oxygen, with the second oil phase being in contact with the alkaline aqueous phase, and obtaining the reaction solution containing the alkylbenzene hydroperoxide. The second oil phase obtained in the water-washing step contains the alkylbenzene, and the alkylbenzene is oxidized to generate the alkylbenzene hydroperoxide in the oxidation step. Such a method for oxidizing the alkylbenzene is described in PTL 1 and the like. For example, if the alkylbenzene contained in the solution used in the alkali extraction step in the present invention is:
(1) o-diisopropylbenzene, o-DHPO can be obtained as main alkylbenzene hydroperoxide;
(2) m-diisopropylbenzene, m-DHPO can be obtained as main alkylbenzene hydroperoxide; and
(3) p-diisopropylbenzene, p-DHPO can be obtained as main alkylbenzene hydroperoxide.

When the second oil phase obtained in the water-washing step contains the alkaline substance, oxidizing the second oil phase with the second oil phase being in contact with the alkaline aqueous phase in the oxidation step refers to further adding an alkaline aqueous solution to the second oil phase and causing the oxidation reaction to occur, or adding only water to the second oil phase and causing the oxidation reaction to occur. When the second oil phase obtained in the water-washing step does not contain the alkaline substance, oxidizing the second oil phase with the second oil phase being in contact with the alkaline aqueous phase in the oxidation step refers to adding an alkaline aqueous solution to the second oil phase and causing the oxidation reaction to occur. The alkaline aqueous solution includes, for example, a sodium hydroxide aqueous solution, a potassium hydroxide aqueous solution, a sodium carbonate aqueous solution and the like, and preferably is a sodium hydroxide aqueous solution. The alkaline aqueous phase has a pH of preferably 7 to 13, and more preferably 8 to 12.

The shape of a reactor is not particularly limited, and a known bubble column or stirring bath may be used. In the present invention, however, a bubble column-type oxidation reactor in which the stirring effect by a gas for oxidation containing air or oxygen can be expected, and particularly an air lift (also called "draft tube-type") oxidation reactor having a partition therein are preferable because oxidation is performed with the second oil phase being in contact with the alkaline aqueous phase. The oxidation reaction temperature is not particularly limited. However, if the temperature is too high, the selectivity of the target alkylbenzene hydroperoxide decreases. On the other hand, if the temperature is too low, the reaction speed is slow, which is not economical. Therefore, the oxidation reaction temperature is 50 to 200°C, and preferably 60 to 120°C. The reaction pressure is not particularly limited. However, if the pressure is too high, energy is required to compress the air or oxygen used in oxidation, which is not economical. On the other hand, if the pressure is too low, the oxygen partial pressure becomes low and a preferable oxidation reaction speed cannot be obtained in some cases, or the rate of bubbles in the reaction apparatus may become high and the effective volume for reaction may become small because the volume of gas becomes larger than the volume of gas when the pressure is high. Therefore, the normal oxidation reaction preferably occurs under pressure, and particularly in the range of 1 to 1000 kPaG.

The reaction solution separation step is the step of performing separation into the third alkaline aqueous phase and the third oil phase containing the alkylbenzene hydroperoxide by oil-water separation of the reaction solution obtained in the oxidation step. The temperature in separating the reaction solution is not particularly limited. However, excessively high temperature is not preferable because the alkylbenzene hydroperoxide decomposes thermally, and excessively low temperature is not preferable because the extraction efficiency may decrease and the solution separation property may become worse. Therefore, the normal water-washing operation temperature is 10 to 80°C, and preferably 20 to 60°C. A container used in the reaction solution separation operation is not particularly limited. The container can, however, include a settling drum having the gravity separation function. In the third alkaline aqueous phase thus obtained, the alkaline substance changes into a salt as a result of neutralization of an organic acid by-produced in oxidation, and the third alkaline aqueous phase has a pH lower than that of the alkaline aqueous solution in the alkali extraction step. Therefore, by recycling at least a part of the water used for water-washing in the water-washing step, the water utilization efficiency in the entire process can be enhanced and the discharged water can be reduced.

In addition to the target alkylbenzene hydroperoxide, the third oil phase obtained in the reaction solution separation step contains alcohol (aromatic alcohol or methanol by-produced in the oxidation step), phenol and an organic acid extracted into the alkaline aqueous solution in the alkali extraction step, and the third oil phase also contains unreacted alkylbenzene to be recycled into the oxidation reaction step as an effective component. Particularly when diisopropylbenzene is used as a material, the third oil phase also contains, as a component to be recycled into the oxidation reaction step, diisopropylbenzene hydroperoxide (more specifically, 2-(2-propyl)-1-(2-hydroperoxy-2-propyl)benzene, 3-(2-propyl)-1-(2-hydroperoxy-2-propyl)benzene, 4-(2-propyl)-1-(2-hydroperoxy-2-propyl)benzene (hereinafter, may be referred to as "MHPO")) or the like, which is an intermediate product obtained as a result of oxidation of only one of the two isopropyl groups. Therefore, it is preferable to recycle the third oil phase obtained in the reaction solution separation step as at least a part of the material solution containing the alkylbenzene, the phenol and the alkylbenzene hydroperoxide, which is used in the alkali extraction step in the present invention, and to perform separation into an alkaline aqueous solution extraction section (containing the target alkylbenzene hydroperoxide as a main component to be extracted) and an oil phase section (component to be recycled).

### 2. Method for manufacturing alkylbenzene hydroperoxide

A method for manufacturing alkylbenzene hydroperoxide according to the present invention includes an alkali extraction step, a water-washing step, an oxidation step, a reaction solution separation step, and a recovery step described below. alkali extraction step: a step of bringing a material solution containing alkylbenzene, phenol and alkylbenzene hydroperoxide into contact with an alkaline aqueous solution, and thereafter, performing separation into a first oil phase containing an alkaline substance and a first alkaline aqueous phase containing at least a part of the phenol and at least a part of the alkylbenzene hydroperoxide
water-washing step: a step of bringing the first oil phase into contact with water, extracting at least a part of the alkylbenzene hydroperoxide and at least a part of the alkaline substance into the water from the first oil phase, and thereafter, performing separation into a second oil phase and a second alkaline aqueous phase containing the alkylbenzene hydroperoxide
oxidation step: a step of oxidizing the second oil phase obtained in the water-washing step, under the presence of an alkaline aqueous phase using a gas containing air or oxygen, and obtaining a reaction solution containing the alkylbenzene hydroperoxide reaction solution separation step: a step of performing separation into a third alkaline aqueous phase and a third oil phase containing the alkylbenzene hydroperoxide by oil-water separation of the reaction solution obtained in the oxidation step recovery step: a step of bringing the second alkaline aqueous phase containing the alkylbenzene hydroperoxide, which was obtained in the water-washing step, into contact with a water-insoluble organic solvent, performing separation into a fourth oil phase containing at least a part of the alkylbenzene hydroperoxide extracted into the water-insoluble organic solvent and an aqueous phase having a reduced concentration of the alkylbenzene hydroperoxide, and discharging the aqueous phase outside the system

The alkylbenzene hydroperoxide includes, for example, ethylbenzene hydroperoxide, alkylbenzene hydroperoxide having at least one 2-hydroperoxy-2-propyl group, and the like. The alkylbenzene hydroperoxide having at least one 2-hydroperoxy-2-propyl group includes, for example, cumene hydroperoxide, o-DHPO, m-DHPO, p-DHPO and the like. The alkylbenzene hydroperoxide is preferably alkylbenzene hydroperoxide having at least one 2-hydroperoxy-2-propyl group, more preferably o-DHPO, m-DHPO or p-DHPO, and further preferably m-DHPO.

If the alkylbenzene hydroperoxide is:
(1) ethylbenzene hydroperoxide, the alkylbenzene is ethylbenzene;
(2) alkylbenzene hydroperoxide having at least one 2-hydroperoxy-2-propyl group, the alkylbenzene is alkylbenzene having at least one isopropyl group;
(3) cumene hydroperoxide, the alkylbenzene is cumene;
(4) o-DHPO, the alkylbenzene is 1,2-diisopropylbenzene;
(5) m-DHPO, the alkylbenzene is 1,3-diisopropylbenzene; and
(6) p-DHPO, the alkylbenzene is 1,4-diisopropylbenzene.

The alkali extraction step, the water-washing step, the oxidation step, and the reaction solution separation step in the method for manufacturing the alkylbenzene hydroperoxide correspond to the steps similar to the alkali extraction step, the water-washing step, the oxidation step, and the reaction solution separation step described in the section about the method for handling the aqueous solution containing the water-soluble substance that changes into the water-insoluble substance over time.

The recovery step is the step of bringing the second alkaline aqueous phase containing the alkylbenzene hydroperoxide, which was obtained in the water-washing step, into contact with the water-insoluble organic solvent, performing separation into the fourth oil phase containing at least a part of the alkylbenzene hydroperoxide extracted into the water-insoluble organic solvent and the aqueous phase having a reduced concentration of the alkylbenzene hydroperoxide as compared with the second alkaline aqueous phase, and discharging the aqueous phase outside the system. Any solvent may be used as the water-insoluble organic solvent used in the recovery step, as long as it allows extraction of the alkylbenzene hydroperoxide contained in the second alkaline aqueous phase and it has a low degree of solubility in water. The water-insoluble organic solvent is preferably ketone having a carbon number of 4 to 10, ether having a carbon number of 4 to 10, or alcohol having a carbon number of 4 to 8, and more preferably methyl isobutyl ketone. The water-insoluble organic solvent may be used alone or in a mixture of two or more types.

From the viewpoint of sufficient extraction of the water-soluble substance that changes into the water-insoluble substance over time as well as reduction in cost such as thermal energy in purifying the solvent, the amount of the water-insoluble organic solvent used is preferably 1 to 200 parts by weight, more preferably 10 to 100 parts by weight, and further preferably 20 to 50 parts by weight with respect to 100 parts by weight of the aqueous solution containing the water-soluble substance that changes into the water-insoluble substance over time.

The recovery temperature is not particularly limited. The normal extraction operation temperature is, however, 10 to 80°C, and preferably 20 to 60°C. A container used in the recovery operation is not particularly limited. The container can, however, include, for example, a combination of a settling drum having the gravity separation function and a portion having the mixing function such as a line mixer and a stirring bath, and the like. When the state of mixed oil and water is good depending on the solution composition, the stirring function may be omitted. In the conventional method, at least a part of the aqueous phase obtained in the water-washing step is discharged without using the recovery step in the present invention. The alkylbenzene hydroperoxide present in the aqueous phase, however, decomposes into alcohol and is precipitated, which causes problems such as blockage in the apparatus or a pipe. It is to be noted that the aqueous phase having a reduced concentration of the alkylbenzene hydroperoxide as compared with the second alkaline aqueous phase due to the recovery step may be treated using the incineration treatment method and the like by the submerged combustion apparatus described in the section about the method for handling the aqueous solution containing the water-soluble substance that changes into the water-insoluble substance over time.

The method for manufacturing the alkylbenzene hydroperoxide according to the present invention is preferably a method for manufacturing the alkylbenzene hydroperoxide wherein the alkylbenzene is alkylbenzene having at least one isopropyl group and the alkylbenzene hydroperoxide is alkylbenzene hydroperoxide having at least one 2-hydroperoxy-2-propyl group, and more preferably a method for manufacturing di(2-hydroperoxy-2-propyl)benzene wherein the alkylbenzene is diisopropylbenzene and the alkylbenzene hydroperoxide is di(2-hydroperoxy-2-propyl)benzene.

### 3. Method for manufacturing hydroxybenzene

A method for manufacturing hydroxybenzene according to the present invention includes a decomposition step described below.
decomposition step: a step of decomposing, under the presence of an acid catalyst, the alkylbenzene hydroperoxide having at least one 2-hydroperoxy-2-propyl group, which was obtained by the above-mentioned method for manufacturing the alkylbenzene hydroperoxide having at least one 2-hydroperoxy-2-propyl group, and obtaining the hydroxybenzene

The alkylbenzene hydroperoxide having at least one 2-hydroperoxy-2-propyl group, whish was obtained by the above-mentioned method for manufacturing the alkylbenzene hydroperoxide having at least one 2-hydroperoxy-2-propyl group, refers to the alkylbenzene hydroperoxide having at least one 2-hydroperoxy-2-propyl group that is contained in the first alkaline aqueous phase obtained in the alkali extraction step in the above-mentioned method for manufacturing the alkylbenzene hydroperoxide having at least one 2-hydroperoxy-2-propyl group. From the viewpoint of using the acid catalyst, it is preferable to perform extraction with a known organic solvent to obtain a decomposition material solution, and remove an alkaline component by water-washing as needed, prior to the decomposition step. From the viewpoint of extraction efficiency and solvent recovery, the organic solvent is preferably ketone having a carbon number of 4 to 10, ether having a carbon number of 4 to 10, or alcohol having a carbon number of 4 to 8, and more preferably methyl isobutyl ketone. The organic solvent may be used alone or in a mixture of two or more types.

Since the alkylbenzene hydroperoxide having at least one 2-hydroperoxy-2-propyl group is used in the decomposition step in the method for manufacturing the hydroxybenzene according to the present invention, acetone is obtained as a by-product and the method for manufacturing the hydroxybenzene according to the present invention is preferable from the viewpoint of industrial implementation.

If the alkylbenzene hydroperoxide having at least one 2-hydroperoxy-2-propyl group that is used in the decomposition step in the method for manufacturing the hydroxybenzene according to the present invention is di(2-hydroperoxy-2-propyl)benzene, the di(2-hydroperoxy-2-propyl)benzene contains the alkylbenzene hydroperoxide of a plurality of types because the di(2-hydroperoxy-2-propyl)benzene is obtained by oxidizing diisopropylbenzene. Therefore, it is preferable to separate desired alkylbenzene hydroperoxide by a known method prior to decomposition. The known method can include a method disclosed in Japanese Patent Laying-Open No. 2005-314269.

Lewis acid such as aluminum chloride, boron trifluoride, ferric chloride and stannic chloride, protonic acid such as sulfuric acid, phosphoric acid, hydrochloric acid, perchloric acid, benzenesulfonic acid, p-toluenesulfonic acid and strong acid ionexchange resin, and the like can be used as the acid catalyst used in the decomposition step. From the viewpoint of yield and ease of handling, the acid catalyst is preferably concentrated sulfuric acid, anhydrous sulfuric acid or fuming sulfuric acid, and more preferably anhydrous sulfuric acid.

The material solution for the decomposition step normally contains 1 to 10 weight % of water although it varies depending on the type of the organic solvent and the type and concentration of the alkylbenzene hydroperoxide. Therefore, the material solution without any change adversely affects the acid catalyst in the decomposition reaction (specifically, decrease in catalyst activity and reduction in decomposition yield). Therefore, it is preferable to distill off a part of the solvent to concentrate the alkylbenzene hydroperoxide prior to the decomposition step. As a result of this concentration operation, the water is also removed. The water is preferably removed to achieve a water concentration of 1 weight % or lower.

The decomposition reaction normally occurs under ordinary or reduced pressure, at a reaction temperature of 30 to 150°C and for a reaction time of 1 to 200 minutes, and an acid decomposition reaction solution is obtained. The decomposition reaction solution contains by-produced acetone, the organic solvent of the decomposition material solution, the acid catalyst, and heavy products, in addition to the hydroxybenzene, e.g., catechol in the case of o-DHPO, resorcin in the case of m-DHPO, and hydroquinone in the case of p-DHPO. It is preferable to cause the decomposition reaction solution to undergo acid catalyst removal by alkali neutralization and solution separation as needed prior to a purification step.

In the method for manufacturing the hydroxybenzene according to the present invention, the purification step described below is preferably performed after the decomposition step.
purification step: a step of purifying the decomposition reaction solution containing the hydroxybenzene, which was obtained in the decomposition step, and obtaining purified dihydroxybenzene

Purification can be performed by using known rectification, crystallization, extraction and the like alone or in combination. Specifically, a method for purifying the dihydroxybenzene can include, for example, a method disclosed in Japanese Patent Laying-Open No. 2002-363118. The hydroxybenzene thus obtained can satisfy the desired quality as industrial products.

### Example

### <Example 1>

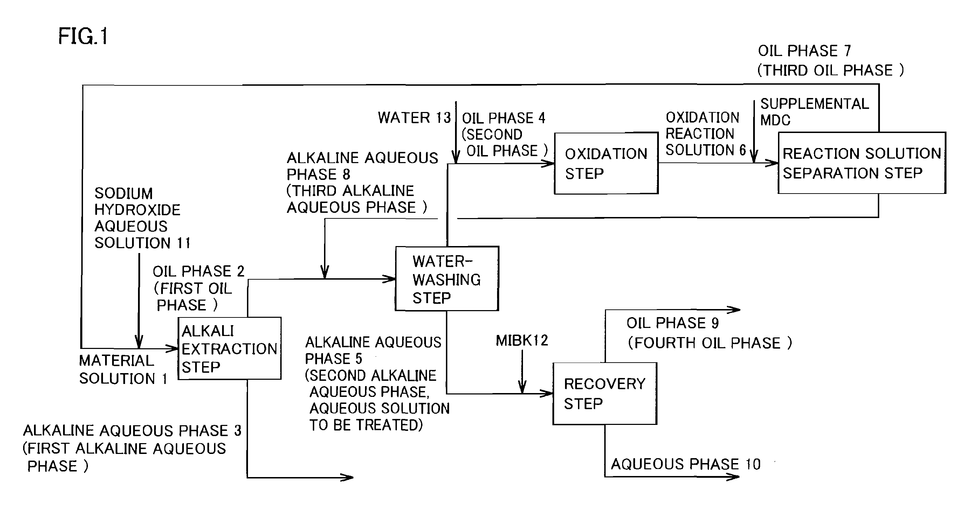

Fig. 1 is a schematic view of a process flow in Example 1 for manufacturing m-DHPO and m-CHPO as the alkylbenzene hydroperoxide.

Alkali extraction step:

A material solution 1 containing m-diisopropylbenzene (hereinafter, may be referred to as "MDC") as alkylbenzene, m-DHPO, m-CHPO and m-MHPO as alkylbenzene hydroperoxide, and isopropylphenol (hereinafter, may be referred to as "m-IPP") as phenol was brought into contact with a sodium hydroxide aqueous solution 11. Thereafter, oil-water separation was performed to obtain an oil phase 2 (first oil phase) containing most of the MDC and the m-MHPO, a part of the m-CHPO, and a small amount of the m-DHPO and an alkaline substance as well as an alkaline aqueous phase 3 (first alkaline aqueous phase) containing most of the m-DHPO, a part of the m-CHPO, and the m-IPP. Alkaline aqueous phase 3 is a product stream containing the m-DHPO and the m-CHPO, which are the target substances in the present invention.

### Water-washing step:

Oil phase 2 obtained in the alkali extraction step was brought into contact with an alkaline aqueous phase 8 (third alkaline aqueous phase) obtained in a reaction solution separation step. Then, oil-water separation was performed to obtain an oil phase 4 (second oil phase) having a reduced concentration of the alkaline substance as well as an alkaline aqueous phase 5 (second alkaline aqueous phase, aqueous solution to be treated) containing the alkaline substance, the m-DHPO and the m-CHPO.

### Oxidation step:

Oil phase 4 having a reduced concentration of the alkaline substance, which was obtained in the water-washing step, was mixed with water 13 and the mixture was oxidized with air. The reaction occurred such that the remaining alkaline substance contained in oil phase 4 was extracted with water 13, and the oxidation reaction occurred with oil phase 4 being in contact with an alkaline aqueous phase. As a result, an oxidation reaction solution 6 was obtained, which was an emulsion of the oil phase and the alkaline aqueous phase, wherein the oil phase mainly contains the target m-DHPO, the m-MHPO serving as an intermediate product, the by-produced m-CHPO and m-IPP, and the unreacted MDC.

### Reaction solution separation step:

Oxidation reaction solution 6 was mixed with a supplemental MDC 14 equivalent to the MDC lost as a result of the reaction. Then, oil-water separation was performed to obtain an oil phase 7 (third oil phase) containing the target m-DHPO, the m-MHPO serving as an intermediate product, the by-produced m-CHPO and m-IPP, and the unreacted MDC, as well as an alkaline aqueous phase 8 (third alkaline aqueous phase). Alkaline aqueous phase 8 was recycled into the water-washing step. Oil phase 7 was recycled into the alkali extraction step.

### Recovery step:

Alkaline aqueous phase 5 obtained in the water-washing step was brought into contact with methyl isobutyl ketone (MIBK) 12 serving as a water-insoluble organic solvent. Thereafter, oil-water separation was performed to obtain discharged water 10 (aqueous phase) having a reduced concentration of the m-DHPO and the m-CHPO as well as an oil phase 9 (fourth oil phase) made of methyl isobutyl ketone (MIBK) that was obtained by recovering the m-DHPO and the m-CHPO. Table 1 shows the composition of alkaline aqueous phase 5 obtained in the water-washing step and supplied to the recovery step. Table 2 shows the composition of discharged water 10 obtained in the recovery step. A stirrer and a settling drum were used as an apparatus for the recovery step, and the temperature was set to approximately 40°C as the operation condition.

As a result, in the method for manufacturing the m-DHPO and the m-CHPO that has the recovery step in the present invention, problems such as blockage in the apparatus did not arise and continuous stable operation for 60 days was possible.

**[Table 1]**

| name of substance | weight % |
|---|---|
| NaOH | 1.42 |
| CHPO | 0.434 |
| DHPO | 0.313 |

**[Table 2]**

| name of substance | weight % |
|---|---|
| NaOH | - |
| CHPO | undetected |
| DHPO | undetected |

### Discharged water 10 obtained in the recovery step in Example 1 was put into a sample bottle and left at room temperature to observe whether crystals of the water-insoluble substance were precipitated or not. As a result, a precipitate was not seen even after a lapse of 30 days.

### <Comparative Example 1>

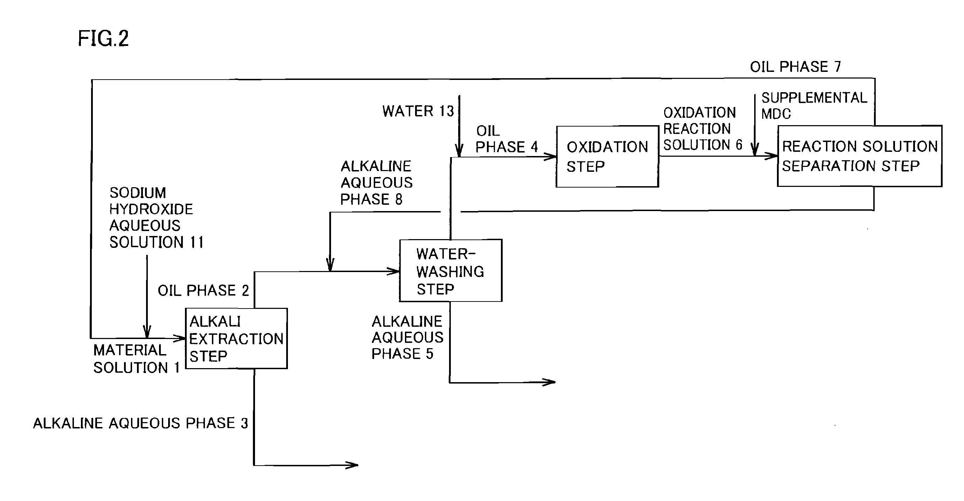

Fig. 2 is a schematic view of a process flow in Comparative Example 1 for manufacturing m-DHPO and m-CHPO as the alkylbenzene hydroperoxide. The process flow was implemented similarly to Example 1 except that the process flow does not have the recovery step. As a result, after a lapse of seven days since the operation started, transport of alkaline aqueous phase 5 obtained in the water-washing step became difficult and continuation of the operation became impossible. As a result of investigation, m-DCA was precipitated, which blocked the pipe.

Alkaline aqueous phase 5 obtained in the water-washing step in Comparative Example 1 was put into a sample bottle and left at room temperature to observe whether crystals of the water-insoluble substance were precipitated or not. As a result, a precipitate was seen after a lapse of three days. This result showed that the problem of crystal precipitation of the water-insoluble substance arises when alkaline aqueous phase 5 is discharged without using the recovery step in the present invention.

### <Example 2>

100 parts by weight of an aqueous solution containing 0.44% of CHPO and 0.29% of DHPO, which serve as the water-soluble substances that change into the water-insoluble substances over time, was brought into contact with 100 parts by weight of MIBK serving as the water-insoluble organic solvent, oil-water separation was performed, and an analysis was performed on the composition of an obtained aqueous phase. As a result, the CHPO in the aqueous phase decreased to a detection lower limit value or smaller, and the DHPO decreased to 0.07%.

### <Example 3>

100 parts by weight of the aqueous solution having the same composition as that in Example 2 was brought into contact with 60 parts by weight of MIBK serving as the water-insoluble organic solvent, oil-water separation was performed, and an analysis was performed on the composition of an obtained aqueous phase. As a result, the CHPO in the aqueous phase decreased to 0.05% and the DHPO decreased to 0.07%.

### <Example 4>

100 parts by weight of the aqueous solution having the same composition as that in Example 2 was brought into contact with 20 parts by weight of MIBK serving as the water-insoluble organic solvent, oil-water separation was performed, and an analysis was performed on the composition of an obtained aqueous phase. As a result, the CHPO in the aqueous phase decreased to 0.10% and the DHPO decreased to 0.14%.

### INDUSTRIAL APPLICABILITY

The present invention can be used in a method for handling an aqueous solution containing a water-soluble substance that changes into a water-insoluble substance over time, which has such excellent features that prevention of problems such as blockage in an apparatus caused by precipitation of the water-insoluble substance as well as long-term stable operation are possible.

### REFERENCE SIGNS LIST

1 material solution; 2 oil phase (first oil phase) obtained in alkali extraction step; 3 alkaline aqueous phase (first alkaline aqueous phase) obtained in alkali extraction step; 4 oil phase (second oil phase) obtained in water-washing step; 5 alkaline aqueous phase (conventional discharged water, second alkaline aqueous phase) obtained in water-washing step; 6 reaction solution obtained in oxidation step; 7 oil phase (third oil phase) obtained in reaction solution separation step; 8 alkaline aqueous phase (third alkaline aqueous phase) obtained in reaction solution separation step; 9 oil phase (fourth oil phase) obtained in recovery step; 10 aqueous phase (discharged water in the present invention) obtained in recovery step; 11 sodium hydroxide aqueous solution; 12 methyl isobutyl ketone (MIBK); 13 water supplied to oxidation step; 14 supplemental MDC

## Claims

1. A method for handling an aqueous solution containing a water-soluble substance that changes into a water-insoluble substance over time, comprising the step of bringing the aqueous solution to be treated containing the water-soluble substance that changes into the water-insoluble substance over time into contact with a water-insoluble organic solvent, and extracting said water-soluble substance into the water-insoluble organic solvent.

2. The method according to claim 1, wherein
said water-soluble substance is alkylbenzene hydroperoxide, and
said water-insoluble substance is alkylbenzene hydroperoxide-derived alcohol.

3. The method according to claim 2, wherein
the alkylbenzene hydroperoxide is 3-(2-hydroxy-2-propyl)-1-(2-hydroperoxy-2-propyl)benzene, and
the alkylbenzene hydroperoxide-derived alcohol is 1,3-di-(2-hydroxy-2-propyl)benzene.

4. The method according to claim 1, wherein
said aqueous solution to be treated is the aqueous solution obtained in a process of manufacturing alkylbenzene hydroperoxide,
said process of manufacturing has:
an alkali extraction step of bringing a material solution containing alkylbenzene, phenol and the alkylbenzene hydroperoxide into contact with an alkaline aqueous solution, and thereafter, performing separation into a first oil phase containing an alkaline substance and a first alkaline aqueous phase containing at least a part of the phenol and at least a part of the alkylbenzene hydroperoxide;
a water-washing step of bringing said first oil phase into contact with water, extracting at least a part of the alkylbenzene hydroperoxide and at least a part of the alkaline substance into the water from said first oil phase, and thereafter, performing separation into a second oil phase and a second alkaline aqueous phase containing the alkylbenzene hydroperoxide;
an oxidation step of oxidizing said second oil phase using a gas containing air or oxygen, with said second oil phase being in contact with an alkaline aqueous phase, and obtaining a reaction solution containing the alkylbenzene hydroperoxide; and
a reaction solution separation step of performing separation into a third alkaline aqueous phase and a third oil phase containing the alkylbenzene hydroperoxide by oil-water separation of said reaction solution, and
said aqueous solution to be treated is said second alkaline aqueous phase.

5. The method according to claim 4, wherein
said water used in said water-washing step contains said third alkaline aqueous phase.

6. The method according to claim 4, wherein
said material solution contains said third oil phase.

7. The method according to claim 4, wherein
said material solution contains diisopropylbenzene as the alkylbenzene and 1,3-di-(2-hydroperoxy-2-propyl)benzene as the alkylbenzene hydroperoxide,
said water-soluble substance is 3-(2-hydroxy-2-propyl)-1-(2-hydroperoxy-2-propyl)benzene, and
said water-insoluble substance is 1,3-di-(2-hydroxy-2-propyl)benzene.

8. A method for manufacturing alkylbenzene hydroperoxide, having:
an alkali extraction step of bringing a material solution containing alkylbenzene, phenol and alkylbenzene hydroperoxide into contact with an alkaline aqueous solution, and thereafter, performing separation into a first oil phase containing an alkaline substance and a first alkaline aqueous phase containing at least a part of the phenol and at least a part of the alkylbenzene hydroperoxide;
a water-washing step of bringing said first oil phase into contact with water, extracting at least a part of the alkylbenzene hydroperoxide and at least a part of the alkaline substance into the water from said first oil phase, and thereafter, performing separation into a second oil phase and a second alkaline aqueous phase containing the alkylbenzene hydroperoxide;
an oxidation step of oxidizing said second oil phase using a gas containing air or oxygen, with said second oil phase being in contact with an alkaline aqueous phase, and obtaining a reaction solution containing the alkylbenzene hydroperoxide;
a reaction solution separation step of performing separation into a third alkaline aqueous phase and a third oil phase containing the alkylbenzene hydroperoxide by oil-water separation of said reaction solution; and
a recovery step of bringing said second alkaline aqueous phase into contact with a water-insoluble organic solvent, performing separation into an aqueous phase and a fourth oil phase containing at least a part of the alkylbenzene hydroperoxide extracted into the water-insoluble organic solvent, and discharging the aqueous phase outside a system.

9. The manufacturing method according to claim 8, wherein
said water used in said water-washing step contains said third alkaline aqueous phase.

10. The manufacturing method according to claim 8, wherein
said material solution contains said third oil phase.

11. The manufacturing method according to claim 8, wherein
the alkylbenzene is alkylbenzene having at least one isopropyl group, and
the alkylbenzene hydroperoxide is alkylbenzene hydroperoxide having at least one 2-hydroperoxy-2-propyl group.

12. A method for manufacturing hydroxybenzene, having a decomposition step of decomposing, under presence of an acid catalyst, alkylbenzene hydroperoxide having at least one 2-hydroperoxy-2-propyl group obtained by the manufacturing method as recited in claim 11, and obtaining the hydroxybenzene.
